# EUROPEAN PATENT SPECIFICATION

(11) **EP 1 439 818 B1**
(45) Date of publication and mention of the grant of the patent: **29.12.2004**
(21) Application number: 02774744.3
(22) Date of filing: 23.10.2002
(51) Int. Cl.: A61K 9/00, A61K 31/195, A61K 33/10, A61K 31/70, A61K 31/198, A61K 33/00

(54) **CHEWABLE TABLET CONTAINING LYSINE**
LYSIN ENTHALTENDE KAUTABLETTE
COMPRIME A MACHER CONTENANT DE LA LYSINE

(30) Priority: 23.10.2001 DE 10152169
(43) Date of publication of application: 28.07.2004
(73) Proprietor: Boehringer Ingelheim International GmbH, 55216 Ingelheim am Rhein (DE)
(72) Inventor: MAROCCHI, Michael, David, DELTA, British Columbia V4K 2S7 (CA); CAPPELLINI, Claudia, CH-6948 PORZA (CH); GIANESELLO, Valter, CH-6945 ORIGLIO (CH)
(86) International application number: PCT/EP2002/011843
(87) International publication number: WO 2003/035027

(56) References cited:
- EP-A- 1 219 182
- FR-A- 2 215 950
- US-A- 2 887 437
- US-A- 3 970 750
- US-A- 5 432 160

## Description

### FIELD OF THE INVENTION

The present invention relates to a tablet with enhanced compliance by humans, in particular children and/or juveniles comprising at least one vitamin, optionally at least one mineral and lysine or a pharmaceutically acceptable salt thereof

### BACKGROUND OF THE INVENTION

It is known that lysine as an essential amino acid enhances appetite and, together with Vitamin D3, improves the absorption of Calcium. The prior art dealing with lysine as nutritional supplement may be best illustrated by the following references:
Albanese A.A. et al., NY State J. Med. 1955; 55, 3453-3456 describe lysine supplementation in infant feeding. Graham G.G. et al., Am. J. Clin. Nutr. 1969; 22 (11), 1459-1468 describe the effect of lysine enrichment of wheat flour for the evaluation in infants. Civitelli R. et al., Nutrition 1992; 8 (6), 400-405, disclose the metabolism of (L)-lysine and calcium in humans. Fürst P., Nutrition 1993; 9 (1), 71-72 suggests (L)-lysine as a nutritional tool in the prophylaxis and treatment of osteoporosis. Flodin N. W., J. Am. Coll. Nutr. 1997; 16 (1), 7-21, reviews the metabolic roles, the pharmacology and the toxicology of lysine.

Accordingly, there is a need to provide humans, in particular children and/or juveniles with lysine supplementation. However, children will hardly accept chewable tablets which contain effective amounts of lysine due to its disgusting taste. The problem underlying the present invention was to provide a lysine containing chewable tablet which is well accepted by children and/or juveniles.

US-A-2 887 437 discloses a tablet which is designed to stimulate growth in children and can be chewed without objectionable taste. Said tablet comprises L-lysine monohydrochloride, several vitamins (B12, B1 nitrate, B6 hydrochloride), sweeters and flavouring agents to mask the taste of lysine.

### SUMMARY OF THE INVENTION

The invention relates to a tablet with enhanced compliance by humans comprising the following constituents:
(a) at least one vitamin,
(b) optionally at least one mineral, selected from the group consisting of manganese (II) gluconate, copper (II) carbonate, calcium phosphate, ferrous (II) fumarate, zinc oxide and magnesium oxide,
(c) lysine or a pharmaceutically acceptable salt thereof,
(d) at least one sweetener and optionally at least one flavoring agent having the capability of masking the disgusting flavor of lysine,
(e) and a pharmaceutically or dietetically acceptable carrier.

Another aspect of the present invention is a method of improving the physiological state of humans which method comprises administering an effective amount of the tablet according to the present invention.

Furthermore, the invention relates to a method for the manufacture of a tablet according to the present invention which method comprises mixing of the different components (a) to (e) and tabletting by direct compression.

### DETAILED DESCRIPTION OF THE INVENTION

The invention relates in particular to tablets to be sucked or chewed by children and/or juveniles wherein the disgusting taste caused by lysine is masked.
Specifically the subject matter of this invention provides tablets intended for the oral way, to be sucked or to be chewed, containing as active ingredients lysine, one or more vitamins and optionally one or more minerals. Lysine is preferably provided in form of a pharmaceutically acceptable salt, in particular as (L)-lysine monohydrochloride. Most preferably the tablet comprises 10 to 100 mg, in particular about 50 mg of (L)-lysine monohydrochloride per unit dosage.

Preferably component (a) comprises at least one vitamin selected from the group consisting of retinol equivalents such as β-carotene or vitamin A, vitamin B such as vitamin B1, vitamin B2, vitamin B6 or vitamin B12, vitamin C, vitamin D such as vitamin D3, vitamin E, folic acid, vitamin H and vitamin PP.

The ranges of amounts of ingredients given hereinabove and hereinbelow relate to the declared amount of said ingredients and include appropriate stability overdosages.

Most preferably component (a) is a mixture of vitamins consisting essentially of 0.4 to 0.8 mg, in particular about 0.5 to 0.7 mg of β-carotene, 500-1500 IU , in particular about 700-1100 IU of vitamin A palmitate, 0.3 to 1.0 mg, in particular about 0.4 to 0.6 mg of vitamin B1 nitrate, 0.3 to 1.0 mg, in particular about 0.5 to 0.7 mg of vitamin B2, 0.3 to 1.0 mg, in particular about 0.5 to 0.7 mg of vitamin B6 hydrochloride, 0.4 to 1.0 µg, in particular about 0.5 to 0.9 µg of vitamin B12, 15 to 40 mg, in particular about 20 to 30 mg of vitamin C, 100-300 IU, in particular about 125-200 IU of vitamin D3, 3.0 to 9.5 mg, in particular about 4.0 to 6.5 mg of vitamin E acetate, 20 to 80 µg, in particular about 40 to 70 µg of folic acid, 10 to 25 µg, in particular about 14 to 21 µg of vitamin H and 4 to 10 mg, in particular about 5 to 8 mg of vitamin PP per unit dosage.

Preferably component (b) comprises at least one mineral selected from the group consisting of manganese such as manganese (II) gluconate, copper such as copper (II) carbonate, calcium such as dicalcium phosphate anhydrous, iron such as ferrous (II) fumarate, zinc such as zinc oxide and magnesium such as magnesium oxide.

Most preferably component (b) is a mixture consisting essentially of 0.2 to 0.8 mg, in particular about 0.4 to 0.6 mg of copper (II) carbonate, 150 to 300 mg, in particular about 200 to 250 mg of dicalcium phosphate anhydrous, 8 to 20 mg, in particular about 11 to 14 mg of ferrous (II) fumarate, 4 to 9 mg, in particular about 6 to 7 mg of zinc oxide and 12 to 28 mg, in particular about 18 to 21 mg of magnesium oxide per unit dosage.

Preferably component (d) contains at least one sweetener selected from the group consisting of calcium saccharinate, ammonium cyclamate, ammonium glycirhizinate, Aspartame (N-*L*-α-aspartyl-*L*-phenylalanine 1-methylester), glucose and glucitols such as inositol, mannitol, sorbitol or dulcitol and at least one flavouring agent selected from the group consisting of natural citrus or orange flavour and Prosweet®, which is a commercially available natural flavouring.

Most preferably component (d) consists essentially of 1.0 to 10.0 mg, in particular 4.0 to 8.0 mg of Aspartame, 100.0 to 400.0 mg, in particular 200.0 to 350.0 mg of glucose, 200 to 800 mg, in particular 300 to 700 mg of sorbitol, 5.0 to 50.0 mg, in particular 10.0 to 30.0 mg of natural orange flavour and 1.0 to 10.0 mg, in particular 2.0 to 6.0 mg of Prosweet® per unit dosage.

Preferably component (e) comprises at least one carrier selected from the group consisting of diluents, excipients, sticking agents, bulk agents, preservatives, colorants and other pharmaceutical or food processing agents.

Among the suitable excipients or diluents, it may particularly be cited acidifying or buffering agents such as citric acid, urea or glycine, bulk agents such or mannitol or sorbitol, adhering agents with low speed of dissolution such as alkyl cellulose, for example methyl cellulose, ethylcellulose, hydroxypropyl cellulose, hydroxypropyl methyl cellulose or carboxy methyl cellulose or copolymers of methacrylic and acrylic acid; binding agents such as silicon dioxide, polyvinyl pyrrolidone, arabic gum, guar gum, adraganth gum, karaya gum, lubricating agents such as magnesium stearate, inert diluents such as lactose, gelatin, starch, mono- or diglyceride fatty acids, edible fatt, sodium aluminium silicate, hydrogenated vegetable oil, calcium carbonate, magnesium phosphate or calcium sulphate; skim milk powder, sodium caseinate.

Among the suitable colorants, it may particularly be cited Turmeric powder (E100), Carmine powder (E120), beta-carotene and Sunset Yellow (E110), Beetroot Red (E162), Erythrosine Red (E127), or a combination of these colorants.

Most preferred is a tablet which can be chewed or sucked with enhanced compliance by children and/or juveniles, preferably at an age of 4 to 16, in particular 6 to 14 years comprising the following constituents:
(a) a mixture of vitamins consisting essentially of 0.4 to 0.8 mg of β-carotene, 500-1500 IU of vitamin A palmitate, 0.3 to 1.0 mg of vitamin B1 nitrate, 0.3 to 1.0 mg of vitamin B2, 0.3 to 1.0 mg of vitamin B6 hydrochloride, 0.4 to 1.0 µg of vitamin B12, 15 to 40 mg of vitamin C, 100-300 IU of vitamin D3, 3.0 to 9.5 mg of vitamin E acetate, 20 to 80 µg of folic acid, 10 to 25 µg of vitamin H and 4 to 10 mg of vitamin PP per unit dosage,
(b) a mixture of minerals consisting essentially of 0.2 to 0.8 mg of copper (II) carbonate, 150 to 300 mg of dicalcium phosphate anhydrous, 8.0 to 20 mg of ferrous (II) fumarate, 4 to 9 mg of zinc oxide and 12-28 mg of magnesium oxide per unit dosage,
(c) 10 to 100 mg of (L)-lysine monohydrochloride per unit dosage,
(d) a mixture of sweeteners and flavoring agents consisting essentially of 1.0 to 10.0 mg of Aspartame, 5.0 to 50.0 mg of glucose syrup, 200 to 800 mg of sorbitol, 5.0 to 50.0 mg of natural orange flavour and 1.0 to 10.0 mg of Prosweet® per unit dosage;
(e) a pharmaceutically or dietetically acceptable carrier,
wherein the complete dosage unit weighs 500 to 2000 mg.

Another aspect of the present invention resides in a method of improving the physiological state of humans, in particular improving the development and growth of children and/or juveniles, most preferably at an age of 4 to 16 years comprising administering orally an effective amount of a tablet comprising the following constituents to said humans:
(a) at least one vitamin,
(b) optionally at least one mineral,
(c) lysine or a pharmaceutically acceptable salt thereof,
(d) at least one sweetener and optionally at least one flavoring agent having the capability of masking the disgusting flavor of lysine,
(e) and a pharmaceutically or dietetically acceptable carrier.

This invention also relates to a process for preparing the tablets according to this invention, which consists in the mixing or conjunction of the active ingredients (a), (b) and (c) with the taste masking agent (d) and with one or several carriers (e) such as diluents, excipients, sticking agents, buffering agents, bulk agents, and/or lubricating agents, to realize a pharmaceutical form suitable to be suckled or chewed, such as tablets, or lozenges. This production is obtained according to the known methods of the pharmacotechny.

The following examples are merely illustrative of the invention without limiting it in any manner.

### EXAMPLE I

Tablets to be sucked

| Component | Function | Declared amount/tablet [mg] |
|---|---|---|
| Active Ingredients (a) + (c) | | |
| (L)-lysine monohydrochloride | essential amino acid | 50.00 |
| Betatab, 10 % (E160a) | vitamin | 5.14 (0.514 β-Carotin) |
| Vitamin A palmitate (500000 IU/g) | vitamin | 1.43 (715 IU) |
| Vitamin B1 nitrate (thiamine mononitrate rocoat 33.3 %) | vitamin | 1.50 (0.50 Vit. B1 nitrate) |
| Vitamin B2 (Riboflavin rocoat 33.3 %) | vitamin | 1.65 (0.55 Vit. B2) |
| Vitamin B6 hydrochloride (Pyridoxin hydrochloride rocoat 33.3 %) | vitamin | 1.65 (0.55 Vit. B6 hydrochloride) |
| Vitamin B12 (Cyanocobolamine 0.1 %) | vitamin | 0.60 (0.60 10⁻³ Vit. B12) |
| Vitamin C (Ascorbic acid 90 %) | vitamin | 24.44 (22.0 Vit. C) |
| Vitamin D3 (Cholecalciferol 100.000 IU/g) | vitamin | 1.50 (150 IU Vit. D3) |
| Vitamin E acetate (50 % d,1-α-tocopherol acetate) | vitamin | 10.43 ( 5.215 Vit. E acetate) |
| Folic acid | vitamin | 0.05 |
| Biotin | vitamin | 1.50 10⁻³(15.0 10⁻³ Vit. H) |
| Vitamin PP (Nicotinamide rocoat 33,3 %) | vitamin | 18.0 (6.0 Vit. PP) |

| Minerals (b) | | |
|---|---|---|
| Copper carbonate | mineral | 0.52 |
| Dicalcium phosphate anhydrous | mineral | 220.64 |
| Ferrous (II) fumarate (coated 60 %) | mineral | 12.68 |
| Zinc oxide (coated 50 %) | mineral | 6.25 |
| Magnesium oxide, heavy | mineral | 19.89 |

| Taste mask (d) | | |
|---|---|---|
| Aspartame powder | sweetener | 6.00 |
| Natural Orange Flavor | flavor | 22.00 |
| Dextrose | sweetener | 275.00 |
| Prosweet® | flavor | 4.00 |
| Sorbitol | sweetener/carrier | 597.44 |

| Carrier (e) | | |
|---|---|---|
| Citric acid | acidifier | 50.00 |
| Silicon dioxide, colloidal | binder | 14.00 |
| Magnesium stearate | lubricant | 12.00 |
| Hydrogenated vegetable oil | diluent | 25.00 |

Once the mixture of components (a), (b), (c) and (d) are perfectly homogenized, the carriers (e) are added thereto. The resulting powder is screened then tabletted by direct compression into tablets having a diameter of 16.0 mm, a thickness of 6.5 to 7.5 mm, a mean weight of about 1400 mg and a hardness of not more than 200 N.

These tablets show a good geometric stability. They swell into an expanded form, practically equal to that of the starting tablet. They progressively and completely release the active ingredients when in contact with saliva in the mouth.

### EXAMPLE II

Determination of the acceptability of the tablets according to this invention.

The acceptability is determined on a group of 144 healthy children (age 6 to 14 years) which received a tablet which corresponds to the recipe of example 1 containing 50 mg (L)-lysine hydrochloride. Each child chewed this tablet until it has been completely consumed. The children are subsequently interviewed about the taste of the product. The same test is then repeated using different multi-vitamin preparations (Prep A and Prep B) presently on the market which do not contain lysine at all.
The following results are obtained:

| | Example 1 (%) | Prep A (%) | Prep B (%) |
|---|---|---|---|
| Observation of behaviour: | | | |
| calmly chews the tablet, it seems to taste well | 52 | 63 | 36 |

| Likeability of the taste: | | | |
|---|---|---|---|
| very good taste | 33 | 45 | 18 |
| good taste | 28 | 23 | 26 |

| Sweetness of tablets: | | | |
|---|---|---|---|
| sweet enough, just right | 70 | 76 | 68 |

| Sourness of tablets: | | | |
|---|---|---|---|
| too sour | 29 | 18 | 39 |
| not too sour | 71 | 82 | 61 |

| Feeling in the mouth after chewing: | | | |
|---|---|---|---|
| good | 76 | 80 | 58 |
| not so good | 24 | 20 | 42 |

| Interest in eating again: | | | |
|---|---|---|---|
| very interested | 20 | 32 | 11 |
| quite interested | 49 | 35 | 35 |

These results clearly show that the tablets according to the present invention despite the high content of (L)-lysine are in the same range of acceptability as Prep A, but are much more acceptable than Prep B.

## Claims

1. A tablet with enhanced compliance by humans comprising the following constituents:
(a) at lesst one vitamin,
(b) at least one mineral selected from the group consisting of manganese (II) gluconate, copper (II) carbonate, calcium phosphate, ferrous (II) fumarate, zinc oxide and magnesium oxide,
(c) lysine or a pharmaceutically acceptable salt thereof,
(d) at least one sweetener and optionally at least one flavoring agent having the capability of masking the disgusting flavor of lysine,
(e) and a pharmaceutically or dietetically acceptable carrier,
wherein said tablet is obtainable by mixing of the different components (a) to (e) and tabletting by direct compression.

2. A tablet according to claim 1, wherein group (a) comprises at least one vitamin selected from the group consisting of β-carotene, vitamin A palmitate, vitamin B1 nitrate, vitamin B2, vitamin B6 hydrochloride, vitamin B12, vitamin C, vitamin D3, vitamin E acetate, folic acid, vitamin H and vitamin PP.

3. A tablet according to claim 1 or 2, wherein group (a) is a mixture of vitamins consisting essentially of 0.4 to 0.8 mg of β-carotene, 500-1500 IU of vitamin A palmitate, 0.3 to 1.0 mg of vitamin B1 nitrate, 0.3 to 1.0 mg of vitamin B2, 0.3 to 1.0 mg of vitamin B6 hydrochloride, 0.4 to 1.0 µg of vitamin B12, 15 to 40 mg of vitamin C, 100-300 IU of vitamin D3, 3.0 to 9.5 mg of vitamin E acetate, 20 to 80 µg of folic acid, 10 to 25 µg of vitamin H and 4 to 10 mg of vitamin PP per unit dosage.

4. A tablet according to any of the preceding claims, wherein group (b) is amixture consisting essentially of 0.2 to 0.8 mg of copper (II) carbonate, 150 to 300 mg of dicalcium phosphate anhydrous, 8.0 to 20 mg of ferrous (II) fumarate, 4 to 9 mg of zinc oxide and 12 to 28 mg of magnesium oxide per unit dosage.

5. A tablet according to any of the preceding claims, wherein group (c) comprises (L)-lysine monohydrochloride.

6. A tablet according to any of the preceding claims, wherein group (c) consists essentially of 10 to 100 mg of (L)-lysine monohydrochloride per unit dosage.

7. A tablet according to any of the preceding claims, wherein group (d) comprises at least one sweetener selected from the group consisting of calcium saccharinate, ammonium cyclamate, ammonium glycirhizinate, Aspartame, glucose and glucitols such as inositol, mannitol, sorbitol or dulcitol and at least one flavouring agent selected from the group consisting of natural citrus or orange flavor and Prosweet®.

8. A tablet according to any of the preceding claims, wherein group (d) consists essentially of 1.0 to 10.0 mg of Aspartame®, 5.0 to 50.0 mg of glucose syrup, 200 to 800 mg of sorbitol, 5.0 to 50.0 mg of natural orange flavor and 1.0 to 10.0 mg of Prosweet® per unit dosage.

9. A tablet according to any of the preceding claims, wherein group (e) comprises at least one carrier selected from the group consisting of diluents, excipients, sticking agents, buffering agents, bulk agents, lubricating agents and colorants.

10. A tablet according to any of the preceding claims which can be chewed or sucked with enhanced compliance by children and/or juveniles comprising the following constituents:
(a) a mixture of vitamins consisting essentially of 0.4 to 0.8 mg of β-carotene, 500-1500 IU of vitamin A palmitate, 0.3 to 1.0 mg of vitamin B1 nitrate, 0.3 to 1.0 mg of vitamin B2, 0.3 to 1.0 mg of vitamin B6 hydrochloride, 0.4 to 1.0 µg of vitamin B12,15 to 40 mg of vitamin C, 100-300 IU of vitamin D3, 3.0 to 9.5 mg of vitamin E acetate, 20 to 80 µg of folic acid, 10 to 25 µg of vitamin H and 4 to 10 mg of vitamin PP per unit dosage,
(b) a mixture of minerals consisting essentially of 0.2 to 0.8 mg of copper (II) carbonate, 150 to 300 mg of dicalcium phosphate anhydrous, 8.0 to 20 mg of ferrous (II) fumarate, 4 to 9 mg of zinc oxide and 12 to 28 mg of magnesium oxide per unit dosage,
(c) 10 to 100 mg of (L)-lysine monohydrochloride per unit dosage,
(d) a mixture of sweeteners and flavoring agents consisting essentially of 1.0 to 10.0 mg of Aspartame, 5.0 to 50.0 mg of glucose syrup, 200 to 800 mg of sorbitol, 5.0 to 50.0 mg of natural orange flavor and 1.0 to 10.0 mg of Prosweet® per unit dosage;
(e) a pharmaceutically or dietetically acceptable carrier,
wherein the complete dosage unit weighs 500 to 2000 mg.

11. Use of a tablet according to claim 1 to 10 for the preparation of a nutritional supplement for improving the physiological state of humans.

12. Use of a tablet according to claim 1 to 10 for the preparation of a nutritional supplement for improving the development and growth of children and/or juveniles.

## Patentansprüche

1. Tablette mit verbesserter Compliance bei Menschen, umfassend die folgenden Bestandteile:
(a) mindestens ein Vitamin,
(b) mindestens ein Mineral ausgewählt aus der Gruppe bestehend aus Mangan(II)gluconat, Kupfer(II)carbonat, Calciumphosphat, Eisen(II)-fumarat, Zinkoxid und Magnesiumoxid,
(c) Lysin oder ein pharmazeutisch annehmbares Salz davon,
(d) mindestens ein Süßungsmittel und gegebenenfalls mindestens einen Geschmacksstoff mit der Fähigkeit, den widerlichen Geschmack von Lysin zu maskieren,
(e) und einen pharmazeutisch oder diätetisch annehmbaren Träger,
wobei die Tablette durch Mischen der verschiedenen Komponenten (a) bis (e) und Tablettieren durch Direktverpressung erhältlich ist.

2. Tablette nach Anspruch 1, worin die Gruppe (a) mindestens ein Vitamin ausgewählt aus der Gruppe bestehend aus β-Carotin, Vitamin A-palmitat, Vitamin B1-nitrat, Vitamin B2, Vitamin B6-hydrochlorid, Vitamin B12, Vitamin C, Vitamin D3, Vitamin E-acetat, Folsäure, Vitamin H und Vitamin PP umfasst.

3. Tablette nach Anspruch 1 oder 2, worin Gruppe (a) eine Mischung von Vitaminen ist, die im wesentlichen aus 0,4 bis 0,8 mg β-Carotin, 500 bis 1.500 IU Vitamin A-palmitat, 0,3 bis 1,0 mg Vitamin B1-nitrat, 0,3 bis 1,0 mg Vitamin B2, 0,3 bis 1,0 mg Vitamin B6-hydrochlorid, 0,4 bis 1,0 µg Vitamin B12, 15 bis 40 mg Vitamin C, 100 bis 300 IU Vitamin D3, 3,0 bis 9,5 mg Vitamin E-acetat, 20 bis 80 µg Folsäure, 10 bis 25 µg Vitamin H und 4 bis 10 mg Vitamin PP pro Dosierungseinheit besteht.

4. Tablette nach irgendeinem der vorhergehenden Ansprüche, worin Gruppe (b) eine Mischung ist, die im wesentlichen aus 0,2 bis 0,8 mg Kupfer(II)carbonat, 150 bis 300 mg Dicalciumphosphat wasserfrei, 8,0 bis 20 mg Eisen(II)fumarat, 4 bis 9 mg Zinkoxid und 12 bis 28 mg Magnesiumoxid pro Dosierungseinheit besteht.

5. Tablette nach irgendeinem der vorhergehenden Ansprüche, worin Gruppe (c) (L)-Lysinmonohydrochlorid umfasst.

6. Tablette nach irgendeinem der vorhergehenden Ansprüche, worin Gruppe (c) im wesentlichen aus 10 bis 100 mg (L)-Lysinmonohydrochlorid pro Dosierungseinheit besteht.

7. Tablette nach irgendeinem der vorhergehenden Ansprüche, worin Gruppe (d) mindestens ein Süßungsmittel ausgewählt aus der Gruppe bestehend aus Calciumsaccharinat, Ammoniumcyclamat, Ammoniumglycyrrhizinat, Aspartam, Glucose und Gluciten, wie Inosit, Mannit, Sorbit oder Dulcit, und mindestens einen Geschmacksstoff ausgewählt aus der Gruppe bestehend aus natürlichem Citrus- oder Orangenaroma und Prosweet® umfasst.

8. Tablette nach irgendeinem der vorhergehenden Ansprüche, worin Gruppe (d) im wesentlichen aus 1,0 bis 10,0 mg Aspartam®, 5,0 bis 50,0 mg Glucosesirup, 200 bis 800 mg Sorbit, 5,0 bis 50,0 mg natürlichem Orangenaroma und 1,0 bis 10,0 mg Prosweet® pro Dosierungseinheit besteht.

9. Tablette nach irgendeinem der vorhergehenden Ansprüche, worin Gruppe (e) mindestens einen Träger ausgewählt aus der Gruppe bestehend aus Verdünnungsmitteln, Exzipienten, Klebmitteln, Puffersubstanzen, Füllstoffen, Gleitmitteln und farbgebenden Mitteln umfasst.

10. Tablette nach irgendeinem der vorhergehenden Ansprüche, die gekaut oder gelutscht werden kann, mit verbesserter Compliance bei Kindern und/oder Jugendlichen, umfassend die folgenden Bestandteile:
(a) eine Mischung von Vitaminen bestehend im wesentlichen aus 0,4 bis 0,8 mg β-Carotin, 500 bis 1.500 IU Vitamin A-palmitat, 0,3 bis 1,0 mg Vitamin B1-nitrat, 0,3 bis 1,0 mg Vitamin B2, 0,3 bis 1,0 mg Vitamin B6-hydrochlorid, 0,4 bis 1,0 µg Vitamin B12, 15 bis 40 mg Vitamin C, 100 bis 300 IU Vitamin D3, 3,0 bis 9,5 mg Vitamin E-acetat, 20 bis 80 µg Folsäure, 10 bis 25 µg Vitamin H und 4 bis 10 mg Vitamin PP pro Dosierungseinheit,
(b) eine Mischung von Mineralien bestehend im wesentlichen aus 0,2 bis 0,8 mg Kupfer(II)carbonat, 150 bis 300 mg Dicalciumphosphat wasserfrei, 8,0 bis 20 mg Eisen(II)fumarat, 4 bis 9 mg Zinkoxid und 12 bis 28 mg Magnesiumoxid pro Dosierungseinheit,
(c) 10 bis 100 mg (L)-Lysinmonohydrochlorid pro Dosierungseinheit,
(d) eine Mischung von Süßungsmitteln und Geschmacksstoffen bestehend im wesentlichen aus 1,0 bis 10,0 mg Aspartam, 5,0 bis 50,0 mg Glucosesirup, 200 bis 800 mg Sorbit, 5,0 bis 50,0 mg natürlichem Orangenaroma und 1,0 bis 10,0 mg Prosweet® pro Dosierungseinheit,
(e) einen pharmazeutisch oder diätetisch annehmbaren Träger,
wobei die vollständige Dosierungseinheit 500 bis 2.000 mg wiegt.

11. Verwendung einer Tablette nach Anspruch 1 bis 10 für die Herstellung einer Nahrungsmittelergänzung zur Verbesserung der physiologischen Verfassung von Menschen.

12. Verwendung einer Tablette nach Anspruch 1 bis 10 für die Herstellung einer Nahrungsmittelergänzung zur Verbesserung der Entwicklung und des Wachstums von Kindern und/oder Jugendlichen.

## Revendications

1. Comprimé convenant mieux à l'être l'humain, comprenant les constituants suivants :
(a) au moins une vitamine,
(b) au moins un minéral choisi dans le groupe consistant en gluconate de manganèse (II), carbonate de cuivre (II), phosphate de calcium, fumarate ferreux (II), oxyde de zinc et oxyde de magnésium,
(c) de la lysine ou un sel pharmaceutiquement acceptable de celle-ci,
(d) au moins un édulcorant et facultativement un agent aromatisant ayant la capacité de masquer le goût écoeurant de la lysine,
e) et un support pharmaceutiquement ou diététiquement acceptable, dans lequel ledit comprimé est susceptible d'être obtenu en mélangeant les différents composants (a) à (e) et par fabrication de comprimés par compression directe.

2. Comprimé selon la revendication 1, dans lequel le groupe (a) comprend au moins une vitamine choisie dans le groupe consistant en β-carotène, palmitate de vitamine A, nitrate de vitamine B1, vitamine B2, chlorhydrate de vitamine B6, vitamine B12, vitamine C, vitamine D3, acétate de vitamine E, acide folique, vitamine H et vitamine PP.

3. Comprimé selon la revendication 1 ou 2, dans lequel le groupe (a) est un mélange de vitamines consistant essentiellement en 0,4 à 0,8 mg de β-carotène, 500-1500 UI de palmitate de vitamine A, 0,3 à 1,0 mg de nitrate de vitamine B1, 0,3 à 1,0 mg de vitamine B2, 0,3 à 1,0 mg de chlorhydrate de vitamine B6, 0,4 à 1,0 µg de vitamine B12, 15 à 40 mg de vitamine C, 100-300 UI de vitamine D3, 3,0 à 9,5 mg d'acétate de vitamine E, 20 à 80 µg d'acide folique, 10 à 25 µg de vitamine H et en 4 à 10 mg de vitamine PP par forme galénique unitaire.

4. Comprimé selon l'une quelconque des revendications précédentes, dans lequel le groupe (b) est un mélange consistant essentiellement en 0,2 à 0,8 mg de carbonate de cuivre (II), 150 à 300 mg de phosphate de dicalcium anhydre, 8,0 à 20 mg de fumarate de ferreux (II), 4 à 9 mg d'oxyde de zinc et 12 à 28 mg d'oxyde de magnésium par forme galénique unitaire.

5. Comprimé selon l'une quelconque des revendications précédentes, dans lequel le groupe (c) comprend du monochlorhydrate de (L)-Lysine.

6. Comprimé selon l'une quelconque des revendications précédentes, dans lequel le groupe (c) consiste essentiellement en 10 à 100 mg de monochlorhydrate de (L)-Lysine par forme galénique unitaire.

7. Comprimé selon l'une quelconque des revendications précédentes, dans lequel le groupe (d) comprend au moins un édulcorant choisi dans le groupe consistant en saccharinate de calcium, cyclamate d'ammonium, glycirhizinate d'ammonium, Aspartame, glucose et glucitols tels que inositol, mannitol, sorbitol ou dulcitol et au moins un agent aromatisant choisi dans le groupe consistant en arôme naturel d'agrume ou d'orange et Prosweet ®.

8. Comprimé selon l'une quelconque des revendications précédentes, dans lequel le groupe (d) consiste essentiellement en 1,0 à 10,0 mg d'Aspartame ®, 5,0 à 50,0 mg de sirop de glucose, 200 à 800 mg de sorbitol, 5,0 à 50,0 mg d'arôme naturel d'orange et 1,0 à 10,0 mg de Prosweet ® par forme galénique unitaire.

9. Comprimé selon l'une quelconque des revendications précédentes, dans lequel le groupe (e) comprend au moins un support choisi dans le groupe consistant en diluants, excipients, agents collants, agents tampons, agents de charge, agents lubrifiants et colorants.

10. Comprimé selon l'une quelconque des revendications précédentes pouvant être mâché ou sucé et convenant mieux aux enfants et/ou aux adolescents, comprenant les constituants suivants :
(a) un mélange de vitamines consistant essentiellement en 0,4 à 0,8 mg de β-carotène, 500-1500 UI de palmitate de vitamine A, 0,3 à 1,0 mg de nitrate de vitamine B1, 0,3 à 1,0 mg de vitamine B2, 0,3 à 1,0 mg de chlorhydrate de vitamine B6, 0,4 à 1,0 µg de vitamine B12, 15 à 40 mg de vitamine C, 100-300 UI de vitamine D3, 3,0 à 9,5 mg d'acétate de vitamine E, 20 à 80 µg d'acide folique, 10 à 25 µg de vitamine H et 4 à 10 mg de vitamine PP par forme galénique unitaire,
(b) un mélange de minéraux consistant essentiellement en 0,2 à 0,8 mg de carbonate de cuivre (II), 150 à 300 mg de phosphate de dicalcium anhydre, 8,0 à 20 mg de fumarate ferreux (II), 4 à 9 mg d'oxyde de zinc et 12 à 28 mg d'oxyde de magnésium par forme galénique unitaire.
(c) 10 à 100 mg de monochlorhydrate de (L)-lysine par forme galénique unitaire,
(d) un mélange d'édulcorants et d'agents aromatisants consistant essentiellement en 1,0 à 10,0 mg d'aspartame, 5,0 à 50,0 mg de sirop de glucose, 200 à 800 mg de sorbitol, 5,0 à 50,0 mg d'arôme naturel d'orange et 1,0 à 10,0 mg de Prosweet® par forme galénique unitaire ;
(e) un support pharmaceutiquement ou diététiquement acceptable, dans lequel la forma galénique unitaire complète pèse 500 à 2000 mg.

11. Utilisation d'un comprimé selon la revendication 1 à 10 pour la préparation d'un supplément nutritionnel pour améliorer l'état physiologique des humains.

12. Utilisation d'un comprimé selon la revendication 1 à 10 pour la préparation d'un supplément nutritionnel destiné à améliorer le développement et la croissance des enfants et/ou adolescents.
